(19) Europäisches Patentamt
European Patent Office
Office européen des brevets

(11) EP 1 365 022 A1

(12) **EUROPEAN PATENT APPLICATION**
published in accordance with Art. 158(3) EPC

(43) Date of publication:
**26.11.2003 Bulletin 2003/48**

(21) Application number: **02710470.2**

(22) Date of filing: **01.02.2002**

(51) Int Cl.7: **C12N 15/09**, A61K 38/17, A61K 48/00, A61P 9/10, C07K 14/47, C07K 16/18, C12N 1/15, C12N 1/19, C12N 1/21, C12N 5/10, C12Q 1/02, C12Q 1/68, G01N 33/15, G01N 33/50, G01N 33/53, C12P 21/08 // C12Q1:02, C12R1:91

(86) International application number:
**PCT/JP02/00844**

(87) International publication number:
**WO 02/061076 (08.08.2002 Gazette 2002/32)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **01.02.2001 JP 2001025962**

(71) Applicant: **MOCHIDA PHARMACEUTICAL CO., LTD.**
**Shinjuku-ku Tokyo 160-8515 (JP)**

(72) Inventors:
- **NAKAMURA, Yusuke**
  **Yokohama-shi, Kanagawa 225-0011 (JP)**
- **SUGANO, Sumio**
  **Tokyo 167-0052 (JP)**
- **YANO, Takashi**
  **c/o MOCHIDA PHARMACEUTICAL CO. LTD.**
  **Tokyo 160-8515 (JP)**
- **KAWANO, Hiroyuki**
  **MOCHIDA PHARMACEUTICAL CO., LTD.**
  **Shinjuku-ku, Tokyo 160-8515 (JP)**
- **WATANABE, Hisayuki**
  **MOCHIDA PHARMACEUTICAL CO.,LTD**
  **Shinjuku-ku, Tokyo 160-8515 (JP)**

(74) Representative: **Wablat, Wolfgang, Dr.Dr.**
**Patentanwalt,**
**Potsdamer Chaussee 48**
**14129 Berlin (DE)**

(54) **ADIPONECTIN-ASSOCIATED PROTEIN**

(57) A novel adiponectin-like protein having therapeutic and/or preventive effects on arteriosclerosis and a gene encoding this protein are provided.

A protein (a) comprising the amino acid sequence represented by SEQ ID No:3 or a protein (b) comprising an amino acid sequence resulting from deletion, substitution or addition of amino acid(s) in the amino acid sequence of SEQ ID NO:3 and having an activity of inhibiting the proliferation of vascular smooth muscle cells or salts of these proteins; and a DNA encoding the above proteins are provided.

EP 1 365 022 A1

SEQUENCE LISTING

<110> Mochida Pharmaceutical Co., Ltd.
<120> Adiponectin like protein
<130> SAP-683-PCT
<150> JP 2001025962
<151> 2001-02-01
<160> 3
<170> PatentIn Ver. 2.1

<210> 1
<211> 1653
<212> DNA
<213> Homo sapiens
<400> 1
atggcccgag gcgctgaggg aggccgtggg gacgcgggtt ggggcctgcg tggcgccctg 60
gcggccgtgg cgctgctctc ggcgctcaac gctgcgggca cggtgttcgc gctgtgccag 120
tggcgcgggc tgagctcggc gctgcgggct ttggaggcgc agcggggccg ggagcagcgc 180
gaggacagtg ccctgcgctc cttcctggcc gagttgagcc gcgcgccgcg cggggcgtcc 240
gcaccacccc aagacccggc cagctcagct cgcaacaagc gcagccacag cggcgagccc 300
gcgccgcata tccgcgccga gagccatgac atgctgatga tgatgaccta ctccatggtg 360
ccgatccgag tgatggtgga cctgtgcaac agcaccaagg gcatctgcct cacaggacct 420
tctggaccac caggacctcc gggagccggc gggttgccag gacacaacgg attggatgga 480
cagcctggtc ctcagggccc aaaaggagaa aaaggagcaa atggaaaaag aggaaaaatg 540
gggatacctg gagctgcagg aaatccaggg gaaaggggag aaaagggaga ccatggtgaa 600
ctgggcctgc agggaaatga gggcccacca gggcagaagg gagaaaaggg tgacaaagga 660
gatgtgtcca acgacgtgct cctggcaggt gccaaaggtg accaaggccc acccggtcca 720
cctgggcccc caggccctcc aggtcctcca gggcccccctg gaagcagaag agccaaaggc 780
cctcggcagc caagcatgtt caacggccag tgcccaggtg agacttgtgc cataccaaat 840
gatgatacct tggttggaaa agctgatgag aaagccagtg aacaccattc cccacaagca 900
gaatccatga tcacttccat tggaaaccca gtgcaagtac tgaaagtgac agagacattt 960
gggacttgga taagagagtc tgctaacaag agtgatgacc ggatttgggt gacagagcat 1020
ttttcaggca tcatggttaa ggaattcaag gatcagccct cacttctgaa tggcagttac 1080
acgttcatcc accttccata ctatttccat ggctgtgggc acgttgttta caacaactct 1140
ctctactacc acaaaggggg ctctaatacc ctagtgagat ttgaatttgg ccaggaaaca 1200
tcccaaactc tgaagcttga aaatgccttg tattttgatc gaaaatacct tttgcaaat 1260

tccaaaactt acttcaatct agctgtagat gaaaagggcc tttggattat ctatgcgtca 1320
agtgtggacg gctcgagcat tcttgtagca caactggatg agaggacatt ctcagtggtg 1380
caacacgtca ataccacgta ccctaaatcc aaggctggca acgccttcat tgcccgagga 1440
atcctctatg tcacagacac caaagatatg agggtcacat ttgcctttga tttgttagga 1500
gggaaacaga tcaatgcaaa ctttgattta agaacttccc agtctgttct tgccatgtta 1560
gcatacaaca tgagagatca gcatttatat tcatgggaag atggccattt aatgctttat 1620
cctgtgcagt ttttgtcaac taccttaaat cag 1653

<210> 2
<211> 2954
<212> DNA
<213> Homo sapiens
<400> 2
agagcatggc ccgaggcgct gagggaggcc gtggggacgc gggttggggc ctgcgtggcg 60
ccctggcggc cgtggcgctg ctctcggcgc tcaacgctgc gggcacggtg ttcgcgctgt 120
gccagtggcg cgggctgagc tcggcgctgc gggctttgga ggcgcagcgg ggccgggagc 180
agcgcgagga cagtgccctg cgctccttcc tggccgagtt gagccgcgcg ccgcgcgggg 240
cgtccgcacc accccaagac ccggccagct cagctcgcaa caagcgcagc cacagcggcg 300
agcccgcgcc gcatatccgc gccgagagcc atgacatgct gatgatgatg acctactcca 360
tggtgccgat ccgagtgatg gtggacctgt gcaacagcac caagggcatc tgcctcacag 420
gaccttctgg accaccagga cctccgggag ccggcgggtt gccaggacac aacggattgg 480
atggacagcc tggtcctcag ggcccaaaag gagaaaaagg agcaaatgga aaaagaggaa 540
aaatggggat acctggagct gcaggaaatc caggggaaag gggagaaaag ggagaccatg 600
gtgaactggg cctgcaggga aatgagggcc caccagggca gaagggagaa aagggtgaca 660
aaggagatgt gtccaacgac gtgctcctgg caggtgccaa aggtgaccaa ggcccacccg 720
gtccacctgg gcccccaggc cctccaggtc ctccagggcc ccctggaagc agaagagcca 780
aaggccctcg gcagccaagc atgttcaacg gccagtgccc aggtgagact tgtgccatac 840
caaatgatga taccttggtt ggaaaagctg atgagaaagc cagtgaacac cattccccac 900
aagcagaatc catgatcact tccattggaa acccagtgca agtactgaaa gtgacagaga 960
catttgggac ttggataaga gagtctgcta acaagagtga tgaccggatt tgggtgacag 1020
agcatttttc aggcatcatg gttaaggaat tcaaggatca gccctcactt ctgaatggca 1080
gttacacgtt catccacctt ccatactatt tccatggctg tgggcacgtt gtttacaaca 1140
actctctcta ctaccacaaa gggggctcta ataccctagt gagatttgaa tttggccagg 1200
aaacatccca aactctgaag cttgaaaatg ccttgtattt tgatcgaaaa tacctttttg 1260
caaattccaa aacttacttc aatctagctg tagatgaaaa gggcctttgg attatctatg 1320
cgtcaagtgt ggacggctcg agcattcttg tagcacaact ggatgagagg acattctcag 1380

tggtgcaaca cgtcaatacc acgtacccta aatccaaggc tggcaacgcc ttcattgccc 1440
gaggaatcct ctatgtcaca gacaccaaag atatgagggt cacatttgcc tttgatttgt 1500
taggagggaa acagatcaat gcaaactttg atttaagaac ttcccagtct gttcttgcca 1560
tgttagcata caacatgaga gatcagcatt tatattcatg ggaagatggc catttaatgc 1620
tttatcctgt gcagtttttg tcaactacct taaatcagtg atgtgctgca ttcggctccc 1680
ttcagcaaat ttcaggggtt ttctgggacc agttctcccc caacagggaa acttgttttt 1740
ttaacgtcag ccagatattt agaaaataac ctcaaaagtg tttatatggt cagtgagccc 1800
cgcttagtga aatagcaaca gattggaagt tgaaatggct gagatttggt gatctcccca 1860
cagctggctc tgcaagttac ctctttctcc ttgggcctta gtttccccat tggtaatctg 1920
aattggctaa gatgattggg gagattttct gtacctgtag gtaatttggt gattcttggt 1980
ggctgctctt ctcacaactt ttatgtatct gcttctgtcg tttagctttt ttagccacat 2040
gctgaccaaa tttacctttg agttgataag tccagtggct tgagtagtga acccctcagt 2100
gctgacttat atcttgttct ttgaaaaaat gcattgactc tttaagacat ctaaagtatc 2160
acattatcca taatttattg cttttctttg catctgcacc tgccaccaca gaataaccat 2220
taccctcagc tgctgattgg gcagctctga gattagcaaa agccagggac agctacatgt 2280
tcaggttttt tttttttttt tttttttcaa taagctattt tttttctttt cttattttaa 2340
atagagagag agtcttgcta tgtttcccag gctggtcttg aactcctggg gctcaagtga 2400
tcctcctgcc ttggcctccc aaaatgctgg attacaggca tgtgtgcctg gcccaggttt 2460
cttaataaaa cagaatcatg atcttccagg ttccccccag tttctgatca tgttgatttg 2520
tagctgtgga tcatgaacac tgaatcctca gatcactctg acttcttatg cttctcctgt 2580
ggatccacta tcaaagtact aaatgctgtg taagtagacg ttaatctggc tggaaccatg 2640
ggaagcactt tgcagtgttc agaagagagg ctccatttgt ggctattatg tagaactggg 2700
ccagagccag tccattgcct gttttttttaa ataaggtttt actgagcaca gccacactca 2760
tttgtttatg cagtacggcc tgacattgct tttgctctgc aacagcagag tcgagtcatt 2820
gcaacaaaga gcatatggcc ccacagtgcc taaaatattg accagctacc cctttatgga 2880
aaaagattgc tgactcctga taaagaatat aaagtgagcc tgattcttga aaaaatcaaa 2940
aaaaaaaaaa aaaa 2954

<210> 3
<211> 551
<212> PRT
<213> Homo sapiens
<400> 3

Met Ala Arg Gly Ala Glu Gly Gly Arg Gly Asp Ala Gly Trp Gly Leu
1 5 10 15
Arg Gly Ala Leu Ala Ala Val Ala Leu Leu Ser Ala Leu Asn Ala Ala

20 25 30

Gly Thr Val Phe Ala Leu Cys Gln Trp Arg Gly Leu Ser Ser Ala Leu

35 40 45

Arg Ala Leu Glu Ala Gln Arg Gly Arg Glu Gln Arg Glu Asp Ser Ala

50 55 60

Leu Arg Ser Phe Leu Ala Glu Leu Ser Arg Ala Pro Arg Gly Ala Ser

65 70 75 80

Ala Pro Pro Gln Asp Pro Ala Ser Ser Ala Arg Asn Lys Arg Ser His

85 90 95

Ser Gly Glu Pro Ala Pro His Ile Arg Ala Glu Ser His Asp Met Leu

100 105 110

Met Met Met Thr Tyr Ser Met Val Pro Ile Arg Val Met Val Asp Leu

115 120 125

Cys Asn Ser Thr Lys Gly Ile Cys Leu Thr Gly Pro Ser Gly Pro Pro

130 135 140

Gly Pro Pro Gly Ala Gly Gly Leu Pro Gly His Asn Gly Leu Asp Gly

145 150 155 160

Gln Pro Gly Pro Gln Gly Pro Lys Gly Glu Lys Gly Ala Asn Gly Lys

165 170 175

Arg Gly Lys Met Gly Ile Pro Gly Ala Ala Gly Asn Pro Gly Glu Arg

180 185 190

Gly Glu Lys Gly Asp His Gly Glu Leu Gly Leu Gln Gly Asn Glu Gly

195 200 205

Pro Pro Gly Gln Lys Gly Glu Lys Gly Asp Lys Gly Asp Val Ser Asn

210 215 220

Asp Val Leu Leu Ala Gly Ala Lys Gly Asp Gln Gly Pro Pro Gly Pro

225 230 235 240

Pro Gly Pro Pro Gly Pro Pro Gly Pro Pro Gly Pro Pro Gly Ser Arg

245 250 255

Arg Ala Lys Gly Pro Arg Gln Pro Ser Met Phe Asn Gly Gln Cys Pro

260 265 270

Gly Glu Thr Cys Ala Ile Pro Asn Asp Asp Thr Leu Val Gly Lys Ala

275 280 285

Asp Glu Lys Ala Ser Glu His His Ser Pro Gln Ala Glu Ser Met Ile

290 295 300

Thr Ser Ile Gly Asn Pro Val Gln Val Leu Lys Val Thr Glu Thr Phe

305 310 315 320

Gly Thr Trp Ile Arg Glu Ser Ala Asn Lys Ser Asp Asp Arg Ile Trp

325 330 335

Val Thr Glu His Phe Ser Gly Ile Met Val Lys Glu Phe Lys Asp Gln

340 345 350

Pro Ser Leu Leu Asn Gly Ser Tyr Thr Phe Ile His Leu Pro Tyr Tyr

355 360 365

Phe His Gly Cys Gly His Val Val Tyr Asn Asn Ser Leu Tyr Tyr His

370 375 380

Lys Gly Gly Ser Asn Thr Leu Val Arg Phe Glu Phe Gly Gln Glu Thr

385 390 395 400

Ser Gln Thr Leu Lys Leu Glu Asn Ala Leu Tyr Phe Asp Arg Lys Tyr

405 410 415

Leu Phe Ala Asn Ser Lys Thr Tyr Phe Asn Leu Ala Val Asp Glu Lys

420 425 430

Gly Leu Trp Ile Ile Tyr Ala Ser Ser Val Asp Gly Ser Ser Ile Leu

435 440 445

Val Ala Gln Leu Asp Glu Arg Thr Phe Ser Val Val Gln His Val Asn

450 455 460

Thr Thr Tyr Pro Lys Ser Lys Ala Gly Asn Ala Phe Ile Ala Arg Gly

465 470 475 480

Ile Leu Tyr Val Thr Asp Thr Lys Asp Met Arg Val Thr Phe Ala Phe

485 490 495

Asp Leu Leu Gly Gly Lys Gln Ile Asn Ala Asn Phe Asp Leu Arg Thr

500 505 510

Ser Gln Ser Val Leu Ala Met Leu Ala Tyr Asn Met Arg Asp Gln His

515 520 525

Leu Tyr Ser Trp Glu Asp Gly His Leu Met Leu Tyr Pro Val Gln Phe

530 535 540

Leu Ser Thr Thr Leu Asn Gln

545 550

## Description

### FIELD OF THE INVENTION

The present invention relates to a novel protein having an activity similar to that of adiponectin which is a protein having an anti-arteriosclerotic activity, and a gene encoding this protein.

### BACKGROUND OF THE INVENTION

Arteriosclerotic diseases such as ischemic heart disease and cerebral infarction are still taken higher rank in the cause of death. Thus, the overcome of these diseases is a problem to be solved in an aging society. It has been proved in several large-scale clinical trials that hypocholesterolemic agent, typical example of which is a HMG-CoA reductase inhibitor, are effective in the treatment of the above diseases. Since the lowering in risk of death is only a few % on the whole even if the agent is used, however, the treatment with the above agent is not necessarily sufficient. Although various coronary angioplasty for coronary diseases have been attempted, restenosis occurring at high percentages after the coronary angioplasty becomes problematic. Therefore, a fundamental approach for arteriosclerotic diseases has not been established even now.

Adhesion of circulating monocytes to vascular endothelial cells and subsequent differentiation to macrophages and foam cell formation as well as their migration and proliferation beneath an endothelium of vascular medial smooth muscle cells are involved in the development and evolution of arteriosclerosis. The proliferation of vascular smooth muscle cells is one of main causes of restenosis after coronary angioplasty.

Adiponectin was discovered as a secretary protein specifically expressing at high level in adipocytes. It was found in subsequent investigation that adiponectin has an anti-arteriosclerotic activity such that it inhibits the adhesion of monocytes to vascular endothelial cells and the proliferation of the smooth muscle cells. In the connection with diabetes, it is shown in a type II diabetic monkey model that the concentration of adiponectin becomes lower with the progression of insulin resistance and that a blood adiponectin level is increased in correlation with an insulin-stimulated peripheral glucose uptake (M rate) in euglycemic clamp method (DIABETES, Vol. 50, pp. 1126-1133 (2001)). Further it is also shown that the physical concentration of adiponectin improves insulin resistance in type II diabetic mice (db/db mouse and $KKA^Y$ mouse) and fat-defective mice (Nature Medicine, Vol. 7, pp. 941-946 (2001)). And, it is reported that patients with coronary disease and obesity show blood adiponectin concentration lower than that of healthy subjects.

An object of the present invention is to provide a novel adiponectin-like protein and a gene encoding this protein.

### SUMMARY OF THE INVENTION

The present inventors cloned a large number of full length cDNAs from a mRNA from human colon according to an oligocapping method and determined nucleotide sequences of these cDNA clones. As the result, a novel gene (C-COL01191) encoding a novel protein comprising 551 amino acids was cloned.

It became clear that a protein encoded by the above gene (hereinafter referred to the "C-COL01191" protein) has an activity similar to that of adiponectin, i.e. an activity of inhibiting the proliferation of vascular smooth muscle cells. From this fact, it is thought that the "C-COL01191" protein is an adiponectin-like protein having an anti-arteriosclerotic activity. That is, the present invention provide a novel "C-COL01191" protein usable in the preparation of therapeutic or preventive pharmaceutical compositions for arteriosclerosis, and a gene encoding this protein.

<gene C-COL01191>

The present invention provides a gene encoding the "C-COL01191" protein. Although this gene can be isolated and identified from the cDNA library as described above, the gene may be a DNA obtained by cloning using a genetic engineering technique such as a hybridization or a chemical synthetic technique such as a phosphoramidite method based on the sequence as disclosed herein. The form of the gene may be a cDNA, a genomic DNA and a chemically synthesized DNA, however not limited thereto.

The DNA of the present invention may be a single strand DNA. Alternatively, it may bind to a DNA or an RNA having the sequence complementary thereto to form a double- or triple-strand. The DNA may be labeled with an enzyme such as horseradish peroxidase (HRPO); a radioactive isotope; a fluorescent substance; a chemiluminescent substance; and the like.

If the nucleotide sequence of the C-COL01191 is provided, a sequence of an RNA and a sequence of a complementary DNA and RNA are univocally determined. Therefore, it should be understood that the present invention also provides an RNA corresponding to the DNA of the present invention as well as a DNA and an RNA having a sequence complementary to the DNA of the present invention. "DNA" and "polynucleotide" are interchangeably used herein.

The DNA of the present invention also includes a DNA hybridizable with the DNA comprising the nucleotide sequence represented by SEQ ID No. 1 under stringent conditions.

Variations of the nucleotide sequence represented by SEQ ID NO:1 are acceptable as long as they are hybridizable with the DNA comprising the nucleotide sequence represented by SEQ ID NO:1 under stringent conditions and a protein encoded by said DNA maintains an activity of inhibiting the adhesion of monocytes to vascular endothelial cells or inhibiting the proliferation of vascular smooth muscle cells. It should be understood that a DNA sequence partially modified by, for example, the presence of plural codons encoding the same amino acid residue due to the degeneracy of codon; and various artificial treatments such as site-specific mutation, random mutation by treating with a mutagen, mutation, deletion, linkage and the like of the DNA fragment by cleaving with a restriction enzyme are included within the present invention as long as it is hybridizable with the DNA represented by SEQ ID No. 1 under stringent conditions and encodes a protein having an activity of inhibiting the adhesion of monocytes to vascular endothelial cells or inhibiting the proliferation of vascular smooth muscle cells even if its sequence is different from the DNA sequence represented by SEQ ID No. 1.

The DNA mutant is acceptable as long as it has a homology with the DNA sequence represented by SEQ ID No. 1 of at least 70%, preferably at least 80%, more preferably at least 90%. The homology in DNA sequence can be analyzed by BLAST (J. Mol. Evol., Vol. 36, pp. 290-300 (1993); J. Mol. Biol., Vol. 215, pp. 403-410 (1990)). The term "hybridizable" means that a DNA is hybridizable with the nucleic acid represented by SEQ ID No. 1 by southern hybridization under stringent conditions. For example, if a probe labeled with DIG Labeling kit (Cat No. 1175033 of Rosche Diagnostics) is used , the hybridization is conducted in a DIG Easy Hyb solution (Cat No. 1603558 of Rosche Diagnostics) at the temperature of, for example, 32°C (preferably 37°C, more preferably 42°C) and the membrane is washed in, for example, 0.5 x SSC solution (containing 0.1% (w/w) SDS) at 50°C (preferably 65°C) (note: 1 x SSC is 0.15M NaCl and 0.015M sodium citrate).

The DNA comprising the nucleotide sequence represented by SEQ ID NO:1 or its partial fragment is believed to be useful as a specific probe for diseases of certain organs since it is confirmed that its expression is different in each organ such as brain, lung, placenta and the like.

The DNA of the present invention can be used to commercially produce the "C-COL01191" protein. And, the DNA can be used for testing the expression status of the protein of the present invention in a tissue by labeling with an enzyme or the like. That is, an expression amount of mRNA as an index of an expression amount of the protein of the present invention in a cell is confirmed by using the DNA as a probe so that a cell and culturing conditions of the cell suitable for the preparation of the protein of the present invention can be determined. In addition, a disease associated with the protein of the present invention can be diagnosed.

Further, an abnormality or polymorphism on the nucleic acid sequence can be tested and/or diagnosed by any method such as PCR-RFLP (Restriction fragment length polymorphism) method, PCR-SSCP (Single strand conformation polymorphism), sequencing method and the like, using a part of the DNA of the present invention as a primer.

And, the DNA of the present invention can be used in gene therapy of a disease in which the expression or the activity of the protein of the present invention is lost, by introducing the DNA of the present invention into an *in* vivo cells.

The DNA of the present invention is very useful in the preparation of a transformant, the production of a recombinant protein using said transformant and the search for a compound specifically enhancing the expression of the protein.

The transformant of the present invention can be produced according to a method known for those skilled in the art. For example, the DNA of the present invention can be incorporated into a suitable host cell using any one of vectors commercially available or easily obtained by those skilled in the art. Then, the expression of the gene C-COL01191 within the host cell can be suitably controlled by placing the gene C-COL01191 under an expression control gene, typical examples of which are a promoter and an enhancer. This technique is suitable for the production of the "C-COL01191" protein using the transformed host cell as well as the investigation of mechanisms how to control the expression of the gene C-COL01191 and the screening of an agent capable of controlling the expression of the gene.

For example, by contacting any candidates with a cell transformed with the vector including the gene C-COL01191 under suitable conditions, an agent capable of promoting the expression of the gene C-COL01191 can be searched among the candidates or evaluated.

By using the DNA of the present invention in combination with a known technique, a transgenic animal can be produced from a suitable animal such as mouse or the like. The transgenic animal is also used in the search or evaluation as well as the transformant. Especially, the gene or the protein of the present invention is associated with arteriosclerosis, coronary diseases, diabetes, ovarian cancer, autoimmune hepatitis, osteoporosis, Cushing's syndrome, rheumatoid arthritis and the like and therefore compounds obtained by the search using the above transformant or transgenic animal are expected to be effective therapeutic or preventive agents for diseases in which the protein of the present invention is involved such as arterioscrelosis.

<"C-COL01191" protein>

The "C-COL01191" protein encoded by the gene C-COL01191 has a predicted amino acid sequence represented by SEQ ID NO:3. This protein is estimated to be a protein having an anti-arteriosclerotic action since it has an activity of inhibiting the proliferation of vascular smooth muscle cells similar to adiponectin. And, as illustrated in Example 9 determining the concentration of the "C-COL01191" protein in sera of patients suffering from various diseases, patients suffering from coronary diseases, diabetes and the like show values lower than those of healthy subjects and patients suffering from ovarian cancer, autoimmune hepatitis, osteoporosis, Cushing's syndrome and rheumatoid arthritis show values higher than those of healthy subjects, from which the relationship between the "C-COL01191" protein and the above diseases became clear. The fact that patients suffering from cardiovascular diseases show lower "C-COL01191" protein values is similar to adiponectin. Whereas, a disease showing higher adiponectin value has not been known. Therefore, it is believed that the fact that the "C-COL01191" protein value is higher in patients suffering from ovarian cancer, autoimmune hepatitis, osteoporosis, Cushing's syndrome and rheumatoid arthritis is characteristic of the "C-COL01191" protein. Diseases showing higher "C-COL01191" protein value include many diseases associated with bone metabolism. Accordingly, the "C-COL01191" protein of the present invention can be also defined to be a protein showing a blood concentration higher than that of a healthy subject in bone metabolism-associated diseases. The present invention also provides a pharmaceutical composition containing this protein as an active ingredient.

Side chains of amino acid residues which are constitutional elements of a protein are different in terms of hydrophobicity, charge, size and the like, but they are known to have several highly conservative relationships since they do not substantially affect a three-dimensional structure (also called as configuration) of the entire protein. Examples of the substitutions of amino acid residues include glycine (Gly) and proline (Pro); Gly and alanine (Ala) or valine (Val); leucine (Leu) and isoleucine (Ile); glutamic acid (Glu) and glutamine (Gln); aspartic acid (Asp) and asparagine (Asn); cysteine (Cys) and threonine (Thr); Thr and serine (Ser) or Ala; lysine (Lys) and arginine (Arg); and the like. Since Ala, Val, Leu, IIe, Pro, methionine (Met), phenylalanine (Phe), tryptophane (Trp), Gly and Cys are classified as apolar amino acids, they are understood to have similar properties to each other. Non-charged polar amino acids include Ser, Thr, tyrosine (Tyr), Asn and Gin. Acidic amino acids include Asp and Glu. Basic amino acids include Lys, Arg and histidine (His) . Even if the conservation as defined above is lost, many mutants maintaining functions essential for the protein are known for those skilled in the art. Further, in several similar proteins conserved between different species, it is recognized that they maintain essential functions even if several amino acids are deleted or inserted concentratedly or scatteringly.

Accordingly, a mutant protein resulting from substitution, insertion, deletion and/or addition of one or more amino acids in the amino acid sequence represented by SEQ ID No. 3 are included within the scope of the present invention as long as it is a protein functionally equivalent to the "C-COL01191" protein, especially it has an activity of inhibiting the adhesion of monocytes to vascular endothelial cells or inhibiting the proliferation of vascular smooth muscle cells.

The above changes in amino acids are found in the nature such as the diversity in sequence between different species or the so-called gene polymorphism. Further, it can be produced artificially according to a known method for those skilled in the art, for example, mutagenesis using a mutagene such as NTG and site-directed mutagenesis using various recombinant gene techniques. The site and the number of the mutation of amino acids are not particularly limited as long as the resultant mutant protein maintains a transcriptional control activity. The mutation number is generally within several tens of amino acids, preferably within 10 amino acids, more preferably within 1 or several amino acids.

Of course, the above protein may be in the form of salt generally admitted by those skilled in the art. And, in case of a protein having a signal sequence, the protein from which the signal sequence is deleted may function as a mature protein. Therefore, it should be understood that a maturation polypeptide in which a signal sequence is deleted from the protein of the present invention is a substance substantially equivalent to the protein of the present invention.

The protein of the present invention can be used in searching for an agent capable of binding to said protein and an agent capable of controlling the activity of said protein. The thus-searched compounds and the like as well as the protein of the present invention or its partial peptide, or salts thereof are expected to be useful as an effective therapeutic or preventive agent for arteriosclerosis.

<Antibody>

Further, the present invention provides an antibody binding to the "C-COL01191" protein of the present invention. The antibody of the present invention is an antibody specifically recognizing the entire "C-COL01191" protein or its partial peptide, or a salt thereof as an antigen. It includes a monoclonal antibody and/or a polyclonal antibody. And, it may be an antibody belonging to any one of five classes (IgG, IgA, IgM, IgD and IgE) classified by the structure, physical-chemical properties and immunological properties of immunoglobulins or either subclass classified by the type of H chain. Further, it may be a fragment such as F(ab')'2 produced by digesting an immunoglobulin with, for example, pepsin, Fab produced by digesting an immunoglobulin with papain and the like, or a chimera antibody. The antibody is useful in investigation or clinical detection of the "C-COL01191" protein, clinical therapy of diseases in which the "C-COL01191" protein is involved, and the like. And, the antibody can be used for preparing an antibody column useful in the purification of the protein of the present invention and detecting the protein of the present invention in each fraction upon purification.

<Antisense nucleic acid>

The present invention provides the so-called antisense nucleic acid capable of inhibiting the biosynthesis of the "C-COL01191" protein at a nucleic acid level *in* vivo. The antisense nucleic acid means a nucleic acid which binds to DNA or RNA involved in carrying a genetic information during either of a transcription stage from a genome region to a pre-mRNA essential for the production of mRNA encoding the "C-COL01191" protein, a processing stage from the pre-mRNA to a mature mRNA, a stage of passing through a nuclear membrane or a translation stage into a protein so as to affect the normal stream of the transmission of the genetic information and thereby to control the expression of the protein. It may comprises a sequence complementary to the entire nucleotide sequence of the gene C-COL01191 or a sequence complementary to either part of the sequence. Preferably, it is a nucleic acid (including DNA and RNA) comprising a sequence corresponding to or complementary to the nucleotide sequence represented by SEQ ID NO: 1 or 2. When the mRNA transcripted from the genome region contains an intron structure or a non-translated region at 5' or 3'-terminal, an antisense nucleic acid corresponding to or complementary to the sequence of the non-translated region will have functions equivalent to those of the antisense nucleic acid of the present invention.

The antisense nucleic acid of the present invention includes a DNA and an RNA as well as all of derivatives similar to the DNA and the RNA in configuration and functions. Example of the antisense nucleic acid includes a nucleic acid having any other substance bound at 3'- or 5'-terminal, a nucleic acid wherein at least one of bases, sugars and phosphates of the oligonucleotide is substituted or modified, a nucleic acid having a non-naturally occurring base, sugar or phosphate, a nucleic acid having a backbone other than the sugar-phosphate backbone and the like. These nucleic acids are suitable as derivatives, in which at least one of a nuclease resistance, a tissue selectivity, a cell permeability and a binding power is improved. That is, the form of the nucleic acid is not limited as long as the nucleic acid can inhibit the activity and the expression of the "C-COL01191" protein.

And, the antisense nucleic acid having a nucleotide sequence complementary to the nucleotide sequence hybridizable with a loop portion of mRNA forming a stem loop, i.e. the nucleotide sequence of a region forming a stem loop is generally preferable in the present invention. Alternatively, an antisense nucleic acid capable of binding to near a translation initiation codon, a liposome binding site, a capping site and a splicing site, i.e. an antisense nucleic acid having a sequence complementary to that of these sites is also preferable since generally it can be expected to be very effective in inhibiting the expression.

In order to make the above antisense nucleic acid incorporated into a cell and act efficiently, it is preferable that the length of the antisense nucleic acid of the present invention is 15 to 30 bases, preferably 15 to 25 bases, more preferably 18 to 22 bases.

The effect of the antisense nucleic acid of the present invention in inhibiting the expression can be evaluated by a known method, for example, by determining an inhibitory activity during a translation stage using *in vitro* transcription (Ribo max systems; Promega) together with in vitro translation (Rabbit Reticulocyte Lysate Systems; Promega) or an expression amount of a reporter gene such as luciferase and the like using an expression plasmid comprising the reporter gene linked to the DNA containing a 5'-non-translated region, a region near a translational initiation site and a 5'-translation region.

Since the antisense nucleic acid of the present invention can inhibit the expression or the activity of the "C-COL01191" protein, it is expected that the antisense nucleic acid is effective therapeutic or preventive agents for diseases in which the protein of the present invention is involved such as arteriosclerosis and the like. Especially the antisense nucleic acid inhibiting the activity and the expression of the "C-COL01191" protein is believed to be effective for ovarian cancer, autoimmune disease, osteoporosis, Cushing's syndrome and rheumatoid arthritis because in these diseases the increase in blood concentration of the protein of the present invention was observed.

BRIEF DESCRIPTION OF DRAWINGS

Fig. 1 shows the results of SDS-PAGE in Example 3. The right lane represents a molecular weight marker and the left lane represents the "C-COL01191" protein expressed by in vitro translation using the plasmid pIVCOL19.

Fig. 2 shows a standard curve of a sandwich ELISA system using the AD1 antibody.

Fig. 3 shows the results of the assay method of sera of healthy subjects and patients suffering from various diseases (34 diseases) by a sandwich ELISA system using the AD1 antibody.

DETAILED DESCRIPTION OF THE INVENTION

<Nucleic acid>

The method for obtaining the DNA of the present invention from a DNA library includes a method comprising screening a suitable genomic DNA library or cDNA library according to a screening method such as a screening method via hybridization, an immunoscreening method using an antibody and the like, amplifying a clone having the desired DNA and cleaving the DNA with a restriction enzyme or the like. In the screening method via hybridization, the hybridization can be conducted for any cDNA library using the DNA having the nucleotide sequence represented by SEQ ID No. 1 or a part thereof labeled with $^{32}P$ or the like as a probe according to a known method (see, for example, Maniatis, T. et al., Molecular Cloning, a Laboratory Manual, Cold Spring Harbor Laboratory, New York (1982)). The antibody used in the immunoscreening method may be the antibody of the present invention as described below. The novel DNA of the present invention may be also obtained by PCR (Polymerase Chain Reaction) using a genomic DNA library or a cDNA library as a template. PCR is conducted for any DNA library according to a known method (see, for example, Michael, A.I. et al., PCR Protocols, a Guide to Methods and Applications, Academic Press (1990)) using sense and antisense primers prepared based on the nucleotide sequence of SEQ ID NO:1, thereby the DNA of the present invention can be obtained. As the DNA library used in the above methods, a DNA library having the DNA of the present invention is selected and used. Any DNA library can be used as long as it comprises the DNA of the present invention. A commercially available DNA library may be also used. Alternatively, a cDNA library may be constructed according to a known method (see J. Sambrook et al., Molecular Cloning, a Laboratory Manual, 2nd ed., Cold Spring Harbor Laboratory, New York (1989)) by selecting cells suitable for the construction of the cDNA library from cells having the DNA of the present invention.

And, the DNA of the present invention can be prepared based on the sequence as disclosed herein by a chemical synthetic technique such as a phosphoramidite method and the like.

The recombinant vector having the DNA of the present invention may have any form such as a cyclic form or a linear form. The recombinant vector may have any other nucleotide sequence in addition to the DNA of the present invention, if necessary. The other nucleotide sequence includes an enhancer sequence, a promoter sequence, a ribosome binding sequence, a nucleotide sequence used for amplifying the number of copies, a nucleotide sequence encoding a signal peptide, a nucleotide sequence encoding other polypeptide, a polyA addition sequence, a splicing sequence, a replication origin, a nucleotide sequence of the gene acting as a selective marker and the like. One preferable example of the recombinant vector of the present invention is an expression vector.

In the gene recombination, it is possible to add a translational initiation codon or a translational stop codon to the DNA of the present invention using a suitable synthetic DNA adaptor, and to newly produce or delete a suitable restriction site within the nucleotide sequence. This is the technique routinely conducted by those skilled in the art. Such a processing can be suitably and easily conducted based on the DNA of the present invention.

As the vector including the DNA of the present invention, a suitable vector is selected and used depending on the type of a host used. The vector may be a plasmid. Alternatively, various viruses may be used, non-limiting examples of which include bacteriophage, vacuro virus, retro virus, vaccinia virus and the like.

The gene of the present invention can be expressed under the control of a promoter sequence inherent in said gene. Using the expression system, an agent promoting the transcription of the gene of the present invention can be efficiently searched. Any other suitable expression promoter can be used by linking it to the promoter sequence inherent in said gene upstream of the gene of the present invention or replacing it with the promoter sequence. In this case, the promoter may be suitably selected depending on a host or an object of expression. For example, if a host is E. coli, a T7 promoter, a lac promoter, a trp promoter, a λPL promoter or the like can be used. If a host is a yeast, a PH05 promoter, a GAP promoter, an ADH promoter or the like can be used. If a host is an animal cell, a promoter from SV 40, a retro virus promoter or the like can be used. These lists are not exclusive.

A method for introducing the DNA into a vector is known (see J. Sambrook et al., Molecular Cloning, a Laboratory Manual, 2nd ed., Cold Spring Harbor Laboratory, New York (1989)). That is, each of the DNA and the vector is digested with a suitable restriction enzyme and the resultant fragments are ligated with a DNA ligase.

<Protein>

The protein of the present invention can be prepared from various organs naturally expressing said protein. Alternatively, it can be chemically synthesized in a peptide synthesizer (for example, Peptide Synthesizer Model 433A; Applied Biosystems Japan) or it can be produced by recombination method using a suitable host cell selected from prokaryotic cells and eukaryotic cells. However, a genetic engineering technique and a recombinant protein produced thereby are preferable in view of purity.

A host cell to be transformed using the recombinant vector described in the previous section is not limitative. Many cells such as lower cells available in genetic engineering techniques, typical examples of which are E. coli, B. subtilis and S.cerevisiae; and animal cells, typical examples of which are insect cell, COS7 cell, CHO cell and Hela cell, can be used in the present invention.

The transformant of the present invention can be obtained by transforming a suitable host cell using the recombinant vector of the present invention. As the method of introducing the recombinant vector described in the previous section into a host cell, some methods are known such as an electroporation, a protoplast method, an alkali metal method, a calcium phosphate precipitation method, a DEAE dextran method, a microinjection method, a method using virus particles and the like (see "Handbook of Genetic Engineering", Special Issue of Experimental Medicines, published by Yodosha Co., Ltd. (March 20, 1991)). Either method may be used.

For preparing the present protein by a genetic engineering technique, the above transformant is cultured to obtain a culture mixture followed by purifying the protein. The transformant can be cultured according to a standard method. Many textbooks are available, for example, "Experimental Procedures in Microbiology", edited by The Japanese Biochemical Society, published by Tokyo Kagaku Dozin Co., Ltd. (1992)) describing the culture of transformants, for reference.

As a method for purifying the protein of the present invention from the culture mixture, a suitable method is selected among conventional methods for purifying proteins. The conventional methods include salting out, ultrafiltration, isoelectric precipitation, gel filtration, electrophoresis, various affinity chromatographies including ion-exchange chromatography, hydrophobic chromatography and antibody chromatography, chromatofocusing, adsorption chromatography, reverse phase chromatography and the like. If necessary, HPLC systems or the like may be used to conduct several methods in a suitable order.

It is possible to express the protein of the present invention as a fused protein with any other protein or tag such as glutathione S transferase, Protein A, hexahistidine tag, FLAG tag and the like. The thus-expressed fused protein may be separated with a suitable protease such as thrombin, enterokinase and the like. This may be more effective for the preparation of the protein. For purifying the protein of the present invention, conventional methods may be suitably combined. Especially if the protein is expressed in the form of a fused protein, it is preferable to purify according to a method characteristic to such a form.

One of methods for preparing the present protein by a genetic engineering technique is the synthesis of a cell-free system using a recombinant DNA molecule (J. Sambrook et al., Molecular Cloning 2nd ed. (1989)).

As mentioned above, the protein of the present invention can be prepared in the form of a single protein or a fused protein with any other different protein. The form of the protein of the present invention is not limited to them. Further, it is possible to transform the protein of the present invention to various forms. For example, it is thought that the protein can be processed according to various methods known for those skilled in the art such as various chemical modifications on the protein, the binding of the protein to a polymeric substance such as polyethylene glycol and the like, the binding of the protein to an insoluble carrier and the like. And, the presence or absence of glycosylation or the difference in glycosylation degree is found depending on a host cell used. It should be understood that all of the above proteins are included within the scope of the present invention as long as said proteins are proteins functionally equivalent to the "C-COL01191" protein.

As salts of the protein of the present invention, salts of physiologically acceptable acids and bases are used. The salts are those prepared according to a known method in the art, examples of which include salts with inorganic acids (hydrochloric acid, phosphoric acid, hydrobromic acid, sulfuric acid and the like) , salts with organic acids (acetic acid, formic acid, propionic acid, fumaric acid, maleic acid, succinic acid, tartaric acid, citric acid, malic acid, oxalic acid, benzoic acid, methanesulfonic acid, benzenesulfonic acid and the like), salts with non-toxic alkali metals and alkaline earth metals (sodium, potassium, lithium, calcium, magnesium, barium and the like), ammonium salt and the like.

<Transgenic animal>

By using the gene C-COL01191 of the present invention, a transgenic non-human mammalian animal can be produced. The transgenic non-human mammalian animal is also included within the scope of the present invention. The transgenic non-human mammalian animal can be produced according to a routine method conventionally used in the production of transgenic animals (see, for example, "Experimental Manual of Genesis, published by Kodansha Scientific Ltd., edited by Motoya KATSUKI under supervision of Tatsuji NOMURA (1987)). That is, the gene or the recombinant vector of the present invention is introduced into a totipotent cell of a non-human animal to produce subjects and thereafter only a subject in which the gene introduced is incorporated in a genome of a somatic cell is selected.

Specifically, in case of a transgenic mouse, a DNA prepared such that the C-COL01191 gene can be expressed is directly poured into a pronucleic oosperm obtained from a normal C57BLack/6 mouse. More specifically, a construct is prepared by introducing the C-COL01191 gene downstream of a suitable promoter by linking. Thereafter, a linear DNA is obtained by removing the sequence from a prokaryote as much as possible, if necessary. This DNA is directly poured into a pronucleus of the pronucleic oosperm using a fine glass needle.

The oosperm is transplanted in an uterus of another pseudopregnant mouse as an allomother. The pseudopregnant mouse is generally prepared by mating an ICR female mouse with a vasectomized or vasoligated male mouse. A genomic DNA is extracted from a tissue from the transplated embryo and confirmed whether or not the C-COL01191 gene is introduced by PCR or southern blotting, thereby a transgenic mouse is obtained.

The so-called "knock-out mouse" can be produced based on the nucleotide sequence of the C-COL01191 (or a mouse homologous gene of C-COL01191). The term "knock-out mouse" used herein means a mouse in which an endogenous gene encoding the protein of the present invention is knocked out (inactivated). The knock-out mouse can be produced by, for example, a positive-negative selection method via homologous recombination (see, for example, US patent Nos. 5,464,764, 5,487,992 and 5,627,059; Proc. Natl. Acad. Sci. USA, Vol. 86, pp. 8932-8935 (1989); Nature, Vol. 342, pp. 435-438 (1989)). Such a knock-out mouse is one embodiment of the present invention.

Recently, the production of clone animals by nuclear transplantation in medium or large animals became possible. In this connection, transgenic and knock-out animals have been practically produced using this technique. That is, a somatic cell or a germinal cell is subjected to homologous recombination based on the nucleotide sequence of the C-COL01191 (or a homologous gene of C-COL01191 in each animal) in the same way as that applied to ES cells and then a nucleus is obtained from the resultant cell and used to obtain a clone animal. This animal is a knock-out animal in which the C-COL01191 gene (or a homologous gene of C-COL01191 in each animal) is lost. Or, the C-COL01191 gene (or a homologous gene of C-COL01191 in each animal)is introduced in any cell of any animal and then the resultant nucleus is used to obtain a clone animal, thereby a transgenic animal can be produced. Such a knock-out non-human animal and a transgenic non-human animal are one embodiment of the present invention irrespective of its species.

<Antibody>

The antibody of the present invention may be polyclonal or monoclonal. Either antibody can be obtained by referring to a known method (see, for example, "Experimental Procedures in Immunology", edited by Japan Society for Immunology, published by Japan Society for Immunology), as describe below in brief.

For obtaining the novel antibody, an animal is inoculated with the protein of the present invention as an immunizing antigen and if necessary a suitable adjuvant such as Freund's complete adjuvant (FCA), Freund's incomplete adjuvant (FIA) and the like. If necessary, a booster at an interval of 2 to 4 weeks may be conducted. After the booster, blood sample is collected for the preparation of an anti-serum. The protein of the present invention used as an antigen is that obtained in any method as long as it has a purity sufficient to be usable in the preparation of an antibody. A partial polypeptide of the protein of the present invention may be suitably used as an immunizing antigen. If the polypeptide used as an immunizing antigen is a low-molecular weight polypeptide, i.e. a polypeptide comprising about 10 to 20 amino acids, it may be linked to a carrier such as keyhole limpet hemocyanin (KLH) and the like and used as an antigen. Animals to be immunized include those conventionally used in immunological experiments by those skilled in the art such as rat, mouse, rabbit, sheep, horse, chicken, goat, pig, cattle and the like, among which preferably a species capable of producing the desired antibody is selected and used.

A polyclonal antibody can be obtained by purifying the resultant anti-serum. The purification may be conducted by suitably combining known methods such as salting-out, ion exchange chromatography, affinity chromatography and the like.

A monoclonal antibody is obtained as follows: An antibody-producing cell such as a spleen cell, a lymphocyte and the like is taken from an immunized animal. The cell is fused with a myeloma cell strain or the like according to a known method using polyethylene glycol, Sendai virus, an electric pulse or the like to produce a hybridoma. Thereafter, a clone producing an antibody which binds to the protein of the present invention is selected and cultured. By purifying a supernatant of the culture of the selected clone, a monoclonal antibody is obtained. The purification may be conducted by suitably combining known methods such as salting-out, ion exchange chromatography, affinity chromatography and the like.

And, the novel antibody is also obtained by a genetic engineering technique. For example, a mRNA is obtained from a spleen cell or a lymphocyte of an animal immunized with the protein of the present invention or its partial polypeptide or from a hybridoma producing a monoclonal antibody against the protein of the present invention or its partial polypeptide. Based on the thus-obtained mRNA, a cDNA library is constructed. A clone producing the antibody which reacts with the antigen is screened and the thus-screened clone is cultured. The desired antibody can be purified from the culture mixture by combined known methods. When the antibody is used for therapy, a humanized antibody is preferable with respect to immunogenicity. The humanized antibody can be prepared by immunizing a mouse whose immune system has replaced with a human immune system (see, for example, Nat. Genet., Vol. 15, pp. 146-157 (1997)). Alternatively, the humanized antibody can be engineered using hypervariable regions of the monoclonal antibody (Method in Enzymology, Vol. 203, pp. 99-121 (1999)).

<Antisense nucleic acid>

The antisense nucleic acid can be prepared according to a known method (see, for example, edited by Stanley T. Crooke and Bernald Lableu, in Antisense Research and Applications, published by CRC Publisher, Florida (1993)). If DNA and RNA are native, the antisense nucleic acid of the present invention can be obtained by synthesizing in a chemical synthesizer or conducting PCR using the C-COL01191 as a template. Alternatively, a part of derivatives such as methyl phosphonate type and phosphorothioate type can be synthesized in a chemical synthesizer (for example, Expedite Model 8909; Applied Biosystems Japan). Then, such a derivative may be synthesized according to a manual attached to the chemical synthesizer and the thus-synthesized product may be purified by HPLC using a reverse phase chromatography or the like, thereby the antisense nucleic acid can be obtained.

When a part of the DNA and the antisense nucleic acid of the present invention is used as a diagnostic or test probe, they are labeled with a radioisotope, an enzyme, a fluorescent substance, a luminescent substance or the like according to a known method. Subsequently, a DNA or a mRNA is prepared from a specimen according to a known method and it is used as a test substance. This test substance is reacted with the labeled probe and then the labeled probe unreacted is washed to remove. If the test substance contains the gene C-COL01191 or RNA, said probe binds thereto. The presence or absence of the binding formation can be known by using a luminescence, a fluorescent, a radioactivity or the like from the enzyme, a fluorescent substance or a luminescent substance labeled; or a radioisotope as an index.

When the DNA, the antisense nucleic acid or the recombinant vector of the present invention is used in clinical applications, it is preferable to use those having a purity suitable for the use of a medicine according to any pharmaceutically acceptable method.

The DNA, the antisense nucleic acid or the recombinant vector of the present invention may be used by directly dissolving or suspending in a suitable solvent. Alternatively, it may be used after encapsulating in a liposome or incorporating in a suitable vector. If necessary, it may be used in a suitable dosage form such as injections, tablets, capsules, eye drops, creams, suppositories, spray, poultices in which pharmaceutically acceptable adjuvants are added. Examples of the pharmaceutically acceptable adjuvants are a solvent, a base, a stabilizer, a preservative, a solubilizing agent, an excipient, a buffer and the like.

When the DNA, the antisense nucleic acid or the recombinant vector of the present invention is used in the above dosage form, its administration method and its dose can be selected depending on the age and the sex of a patient, the type and severity of the disease. Thus, it may be administered in an amount suitable to improve arteriosclerosis by the suitable method selected from oral, inhalation, transdermal, intravaginal, intraarticular, intrarectal, intravenous, local, intramuscular, subcutaneous and intraperitoneal administrations.

<Screening method>

The present invention relates to a method of screening an agent capable of controlling the function or the expression of the protein of the present invention, which comprises using the protein of the present invention, a transformant expressing said protein, the DNA of the present invention, a recombinant vector comprising said DNA, a transformant produced by transforming with said vector or a transgenic non-human mammalian animal produced by transforming with the DNA of the present invention.

More specifically, the screening method includes:

(1) a method of evaluating an activity of the "C-COL01191" protein in inhibiting the adhesion of monocytes to vascular endothelial cells or inhibiting the proliferation of vascular smooth muscle cells in the presence /absence of a candidate;

(2) a method of screening an agent capable of controlling the expression of the protein of the present invention by comparing an expression level of the protein or the gene of the present invention in the presence /absence of a candidate; and the like. Example of the method (1) is a method comprising determining an activity of the "C-COL01191" protein in the presence /absence of a test substance in a system as illustrated in Example 4. Example of the method (2) is a method comprising determining an expression amount of a reporter gene such as luciferase or the like under the condition where the gene of the present invention is transcripted or translated in the presence/absence of a candidate using an expression plasmid prepared by linking the reporter gene to the DNA containing an expression control region, a 5'-non-translated region, a region near a translational initiation site, a 5'-translation region or the like of the gene of the present invention so as to confirm a transcriptional promotion activity or a transcriptional inhibiting activity of the candidate.

An agent capable of controlling the activity of the protein of the present invention may be an agent capable of either enhancing(agonist) or inhibiting (antagonist) the activity of the "C-COL01191" protein. An agent capable of controlling the expression of the DNA of the present invention may be an agent capable of either promoting or inhibiting the expression of the gene C-COL01191. An agent capable of inhibiting the expression is preferable. For confirming whether a candidate controls the activity of the protein of the present invention or the expression of the DNA of the present invention, a difference in the activity of the protein or the expression level of the DNA is determined between the addition and no addition of a candidate in a system capable of confirming the activity of the protein or the expression of the DNA. The expression level of the DNA is determined on the basis of an expression strength of the gene C-COL01191 into mRNA or the protein. Instead of the expression level of the C-COL01191 gene or the "C-COL01191" protein per se, an expression level of a reporter gene may be detected. The reporter-assay system means an assay method in which an expression amount of a reporter gene arranged downstream of a transcriptional control region is determined to screen an agent affecting the transcriptional control region. Examples of the transcriptional control region include a promoter, an enhancer, a CAAT box, a TATA box and the like generally found in a promoter region. As a reporter gene, a CAT (chloramphenicol acetyl transferase) gene, a luciferase gene, a β-galactosidase gene and the like can be used. The expression control region and the 5'-non-translated region of the gene of the present invention can be obtained according to a known method (see "New Experimental Protocol in Cell Engineering", published by Shojunsha Co., Ltd. (1993)). Having function of inhibiting (or controlling) or enhancing (or promoting) means that a determined value as to the activity of the protein or the expression level of the DNA is different between a group with the addition of a candidate and a group without the addition of a candidate. For example, the inhibition (or control) or the enhancement (or promotion) ratio calculated by the following equation is 10% or higher, preferably 30% or higher, more preferably 50% or higher, even preferably 70% or higher, especially preferably 90% or higher.

inhibition (or control) or enhancement (or promotion) ratio (%) [(determined value of a group without the addition of a candidate) minus (determined value of a group with the addition of a candidate)] / (determined value of a group without the addition of a candidate) x 100

Either inhibition or enhancement is suitably determined depending on the kind of a system capable of confirming the activity of the protein or a system capable of confirming the expression of the DNA. For example, if a system capable of confirming the activity of the protein is a system for determining an activity controlling the proliferation of vascular smooth muscle cells as shown in Example 4, a radioactivity can be used. When the determined value in a group with the addition of a candidate is higher than that in a group without the addition of a candidate, the candidate can be judged to have a function of inhibiting the activity of the "C-COL01191" protein. Of course, if values from background and/or noises is contained in a determination system, they should be subtracted.

Since the protein of the present invention is an adiponectin-like protein, compounds obtained through the search using the screening method or transgenic animal described above are expected to be effective therapeutic or preventive agents for arteriosclerosis. Non-limiting examples of a candidate include proteins, peptides, oligonucleotides, synthetic compounds, naturally occurring compounds, fermented products, cell extracts, plant extracts, animal tissue extracts and the like. The candidate may be either new or known.

<Pharmaceutical composition>

The pharmaceutical composition of the present invention means a pharmaceutical composition comprising the protein of the present invention or its salt as defined above together with a pharmaceutically acceptable carrier. Examples of the pharmaceutically acceptable carrier include an excipient, a diluent, a filler, a disintegrating agent, a stabilizer, a preservative, a buffer, a perfuming agent, a coloring agent, a sweetening agent, a thickener, a flavor, a solubilizing assistant and the like. If necessary, various additives usable in conventional protein preparations such as a stabilizer, a bactericide, a buffer, an isolating agent, a chelating agent, a pH controller, a surfactant and the like may be suitably added.

As unit dosage forms, various forms such as tablets, pills, powders, granules, injections, solutions, capsules, elixirs, syrups, suspensions, emulsions and the like can be selected. These pharmaceutical compositions can be administered orally or parentally.

A dosage of the pharmaceutical composition is not particularly limited. It is suitable determined depending on age, sex, body weight and condition of a patient, a therapeutic effect desired, an administration route and the like.

<Assay method, reagent and kit using the present antibody>

The present invention provides

(1) an assay method for the "C-COL01191" protein in a test sample comprising using the antibody of the present invention;

(2) an assay reagent or kit for the "C-COL01191" protein in a test sample comprising using the antibody of the present invention ;

(3) an assay method for the "C-COL01191" protein as mentioned in (1) which comprises determining the increase or decrease in an amount of the "C-COL01191" protein in a human body fluid to predict, detect or diagnose a dysfunction of the "C-COL01191" protein, diseases accompanied with the dysfuntion or conditions associated with the diseases; and

(4) an assay method as mentioned in (3) wherein the disease is at least one selected from coronary diseases (angina pectoris, acute myocardial infarction), type II diabetes, pulmonary tumor, heart failure, cardiac disease, hypertension, hepatic failure, hepatic cirrhosis, pancreatitis, acute leukemia, chromatic leukemia, brain infarction, Alzheimer's senile dementia, dementia, Sjogren's syndrome, ovarian cancer, autoimmune hepatitis, osteoporosis, Cushing's syndrome and rheumatoid arthritis.

The assay method of the present invention comprises a step using the antibody of the present invention. Preferably this step is a step comprising trapping a test substance, i.e. the "C-COL01191" protein, in a test sample by an antigen-antibody reaction of said test substance with the antibody of the present invention. Principle of detecting a test substance in the assay method of the present invention are not particularly limited, examples of which include an agglutination method, a sandwich method, a solid phase direct method, a solid phase binding method, a competitive method and the like. Among them, a sandwich method and a competitive method are preferable, a sandwich method being especially preferable.

In an agglutination method, an antibody binds to surfaces of particles such as latex particles and erythrocytes (for example, sheep erythrocytes) such that the particles are agglutinated if the "C-COL01191" protein is present. In this method, the "C-COL01191" protein is determined in terms of an agglutination degree of particles. In this agglutination method, conventionally used particles such as gelatin, microbeads, carbon particles and the like can be used in addition to latex particles and erythrocytes.

In a sandwich method, a solid phase direct method, a solid phase binding method and a competitive method, a labeled antibody or antigen is used. The determination can be conducted according to the principles of enzyme immunoassay method (EIA), radioimmunoassay method (RIA), chemiluminescence immunoassay method, fluoroimmunoassay method, time-resolved fluoroimmunoassay method (TR-FIA), immunochromatography assay method and the like.

A sandwich method, a solid phase direct method and a competitive method based on the principle of EIA which is one of the preferable embodiment of the assay method of the present invention will be described below.

In a sandwich method via EIA, an antibody or a secondary antibody which recognizes the "C-COL01191" protein and is labeled with an enzyme such as peroxidase, alkali phosphatase, β-galactosidase or the like is provided. Especially a polyperoxidase-labeled antibody is preferable. And, an antibody recognizing the "C-COL01191" protein is adsorbed on a solid phase to be used. After a sample or a standard is added to the solid phase, the above enzyme-labeled antibody is added so as to conduct an antigen-antibody reaction. Excess enzyme-labeled antibody is removed by washing. Thereafter a chromophoric substrate selected depending on an enzyme used, for example *o*-phenylene-

diamine and $H_2O_2$, p-nitrophenyl phosphoric acid, 2-nitrophenyl-β-galactoside or the like is added to react with the enzyme. Since the substrate is colored depends on an amount of the enzyme and thereby an amount of the "C-COL01191" protein in a sample, the concentration of the "C-COL01191" protein can be quantified by determining an amount of the resultant colored product.

In a solid phase direct method, a sample is directly adsorbed on a solid phase. Surfaces on the solid phase where the "C-COL01191" protein is not adsorbed are blocked with a protein not affecting its assay system, for example BSA (bovine serum albumin) or the like and then an enzyme-labeled antibody recognizing the "C-COL01191" protein is added and reacted. The subsequent procedures are similar to those in the above sandwich method so as to qualitatively or quantitatively determine the "C-COL01191" protein.

In a competitive method, a predetermined amount of the "C-COL01191" protein recognized by an antibody used is directly adsorbed on a solid phase. After the solid phase is blocked, an enzyme-labeled antibody recognizing the "C-COL01191" protein and a sample are added thereto. They are reacted for certain period and the solid phase is washed to remove substances unbound to the solid phase, to which a chromophoric substrate is added to react with the enzyme. After the reaction, the inhibition in binding of the enzyme-labeled antibody to the "C-COL01191" protein onto the solid phase is determined so as to quantify the "C-COL01191" protein in the sample.

The "C-COL01191" protein in a sample may be quantified by adsorbing an antibody onto a solid phase, adding an enzyme-labeled "C-COL01191" protein and a sample simultaneously and then determining the inhibition in binding of the enzyme-labeled product to the "C-COL01191" protein on the solid phase due to the addition of the sample..

Any method other than the above-mentioned assay methods includes an assay method comprising conducting an antigen-antibody reaction in a liquid phase, separating the "C-COL01191" protein bound to a labeled antibody from the "C-COL01191" protein unbound according to an agglutinating precipitation method using an antibody or a physical-chemical technique and then quantifying. Alternatively, it is possible to determine the "C-COL01191" protein by preparing a secondary antibody recognizing an antibody which recognize the "C-COL01191" protein, labeling the secondary antibody and then conducting an antigen-antibody reaction.

In either a sandwich method, a solid direct method or a competitive method, the combination of a labeled enzyme and a chromophoric substrate may be replaced with the combination of a labeled enzyme and a bioluminescent or chemiluminescent substrate or the combination of a labeled enzyme and a fluorescent substrate or the like. Typical examples of the combination of an enzyme and a luminescent substrate include alkali phosphatase-AMPPD, horse radish peroxidase-luminol, luciferase-luciferin and the like. Examples of the combination of an enzyme and a fluorescent substrate include alkali phosphatase-umbelliferyl phosphate, horse radish peroxidase-p-hydroxyphenyl propione and the like.

Further, in the above three assay methods, it is possible to quantify the "C-COL01191" protein in a sample by using an antibody or antigen directly or indirectly labeled with a radioactive substance, a chemiluminescent substance or a fluoroluminescent substance in stead of an enzyme and determining an intensity of radioactivity, luminescence or fluorescence.

As a radioactive substance, $^{125}I$, $^{131}I$ and the like are generally used. Typical chemiluminescent substances include acridium ester and the like. For determining a fluorescent intensity, an assay method comprising directly or indirectly binding a chelating agent to an antibody or an antigen, exposing it to an excitation radiation and determining a fluorescent intensity issued from a rare earth metal bound to the chelating agent with time-resolution so as to quantify the "C-COL01191" protein in a sample is also useful. This method is more sensitive. Typical examples of the rare earth metal include europium.

The object of the assay method of the present invention is to detect or determine the "C-COL01191" protein in a sample. A sample to be tested includes a body fluid, a tissue, a cell or a cell body of an animal (especially human), and their extract, culture supernatant, smear and slice. The body fluid is preferable. More preferable sample is selected from blood, plasma, serum, urine, liquor, lymph, saliva, ascites and pleural effusion.

By using the assay method of the present invention, the determination of the "C-COL01191" protein in body fluids of healthy subjects and patients suffering from different diseases became possible. And, the concentration of the "C-COL01191" protein in body fluids was determined by the present invention. In addition, it became clear that the concentration of the "C-COL01191" protein varies depending on the type of diseases. When the concentration of the "C-COL01191" protein in body fluids of patients suffering from different diseases is compared with those of healthy subjects, the determined values are statically analyzed according to a method conventionally used by those skilled in the art so as to judge whether or not a difference therebetween is significant.

The assay reagent and the assay kit of the present invention can be constituted in accordance with the above assay method.

## EXAMPLES

The present invention will be described in more detail by referring to the following examples which are not to be construed as limiting the scope of the invention.

Example 1: Cloning of C-COL01191 gene

(1) Construction of full length cDNA library according to oligocapping method

A poly (A) +RNA was prepared from a human colon according to the method of Sambrook et al. (Molecular Cloning. A Laboratory Manual, 2nd edition, Cold Spring Harbor Laboratory, Cold Spring Harbor, NY) using an oligo-dT cellulose. Next, 5 to 10 μg of the poly(A) +RNA was reacted with 1.2U of Bacterial Alkaline Phosphatase (hereinafter abbreviated as "BAP") (TaKaRa) in a buffer containing 100mM of Tris-HCl (pH 8.0), 5mM of 2-mercaptoethanol and 100U of RNasin (Promega) at 37°C for 40 minutes so as to dephosphorylate the poly(A)+RNA having no cap structure. Thereafter, the reaction was extracted with a mixture of phenol and chloroform (1:1) twice and the poly(A)+RNA was precipitated with ethanol. The thus-collected poly(A)+RNA was treated with 20U of Tobacco acid pyrophosphatase (hereinafter abbreviated as "TAP") (Maruyama and Sugano, Gene, Vol. 138, pp. 171-174 (1994)) in a buffer containing 50mM of sodium acetate (pH 5.5), 1mM of EDTA, 5mM of 2-mercaptoethanol and 100U of RNsin at 37°C for 45 minutes so as to remove the cap structure. Thereafter, the reaction was extracted with a mixture of phenol and chloroform (1: 1) twice and subjected to ethanol precipitation to collect a BAP-TAP treated poly(A) +RNA.

2 to 4 μg of the thus-collected poly(A) +RNA was ligated with 0.4 μg of 5'-oligomer (5'-AGC AUC GAG UCG GCC UUG UUG GCC UAC UGG-3'). This reaction was conducted with 250U of RNA ligase (Takara Shuzo Co., Ltd.) in a buffer containing 50mM of Tris-HCl (pH 7.5), 5mM of $MgCl_2$, 5mM of 2-mercaptoethanol, 0.5mM of ATP, 25% of PEG8000 and 100U of RNasin at 20°C for 3 to 16 hours. Thereafter, the unreacted oligomer was removed before cDNA synthesization. That is, 2 to 4 μg of the oligocapped poly(A)+RNA was mixed with 10pmol of a dT adapter-primer (5'-GCG GCT GAA GAC GGC CTA TGT GGC CTT TTT TTT TTT TTT TTT-3') and the mixture was reacted with Superscript II RNase $H^-$ Reverse Transcriptase (Gibco BRL) in the provider's buffer at 42°C for 1 hour.

The reaction was conducted with 15mM of NaOH at 65°C for 1 hour to remove the RNA as a template and then a cDNA was amplified. 1 μg of the cDNA synthesized from the oligocapped poly (A) +RNA was mixed with 16pmol of a sense primer-1 (5'-AGC ATC GAG TCG GCC TTG TTG-3') and an antisense primer-1 (5'-GCG GCT GAA GAC GGC CTA TGT-3') and amplified using XL PCR kit (Perkin-Elmer). The reaction condition for PCR was 5 to 10 cycles, each comprising heating at 94°C for 1 minute, at 58°C for 1 minute and at 72°C for 10 minutes. The PCR product was extracted with a mixture of phenol and chloroform (1:1) and collected as ethanol precipitates. Thereafter, the thus-collected product was digested with SfiI and subjected to electrophoresis on agarose gel to separate a cDNA of 1,000 bp or higher. This cDNA was inserted into DraIII site of pME18S-FL3 (GenBank accession No. AB009864) which was an expression vector for mammalian cells. Since this DraIII site of pME18S-FL3 was asymmetrical, the terminal of the cDNA fragment was a SfiI site complementary thereto and therefore the cDNA fragment was inserted unidirectionally.

(2) Sequencing of cDNA clone and analysis of information about its predicted protein

A plasmid was prepared from the cDNA library constructed by the method described in the above section (1) by means of a pI-100 robot (KURABO). Each clone was sequenced and the resultant sequence was used as data base. The sequencing was conducted using AutoCycle sequencing kit (Amersham Pharmacia) and R.O.B. DNA processor (Amersham Pharmacia) according to the provider's protocol in ALF DNA sequencer (Amersham Pharmacia).

The plasmid C-COL01191 obtained by the method (1) contained a cDNA comprising 2954 base pairs represented by SEQ ID NO:2 containing an open reading frame comprising a nucleotide sequence of 1653 base pairs represented by SEQ ID NO:1, encoding a novel protein comprising 551 amino acids represented by SEQ ID NO:3. This plasmid C-COL01191 was deposited in International Patent Organism Depositary (IPDO) (Tsukuba City, Ibaragi Prefecture, Japan) of National Institute of Advanced Industrial Science and Technology as FERM BP-7860 on January 24, 2001.

For searching the homology of the amino acid sequence of the "C-COL01191" protein (551 amino acids) with the amino acid sequence of adiponectin (244 amino acids), BLAST (Altschul SF., J. Mol. Evol., Vol. 36, pp. 290-300 (1993); Altschul SF. et al, J. Mol. Biol., Vol. 215, pp. 403-10 (1990)) was conducted to search a local agreement in sequences. As the result, a region of 74 amino acid residues from amino acid Nos. 33 to 106 and a region of 64 amino acid residues from amino acid Nos. 42 to 105 of adiponectine were found in the "C-COL01191" protein at the homology of 42%, respectively. And, it became clear from the prediction of a signal peptide via sigclave that a signal sequence is present in the amino acid sequence of amino acid Nos. 19 to 32 in the amino acid sequence represented by SEQ ID NO:3. These facts suggested that the "C-COL01191" protein is a secretory protein similar to adiponectin.

Example 2: Analysis of expression in various organs by PCR method

In order to evaluate the expression of the gene C-COL01191 in various organs, PCR was conducted using a commercial available cDNA (Human MTC Panel I and II) (CLONTECH) as a template and specific sense primer (5'-GGC TTG AGT AGT GAA CCC CT-3') and antisense primer (5'-AAG CAA TGT CAG GCC GTA CT-3') which were prepared based on the sequence in 3'-non- translated region of the sequence of the gene C-COL01191 by PLATINUM Taq DNA Polymerase (Gibco BRL). PCR was conducted by reacting the reaction liquid at 94 for 2 minutes and then subjecting to 30 cycles, each cycle comprising heating at 94°C for 30 seconds, at 55°C for 30 seconds and at 72°C for 1 minute. As the result, the expression of the gene C-COL01191 was found in brain, lung and placenta. Especially significant expression was found in placenta. While, no expression was found in thymus, spleen, peripheral blood lymphocyte, heart, liver, kidney, pancreas, colon, small intestine, skeletal muscle, prostate, testis and ovary.

Example 3 : Expression of protein by *in vitro* translation

In order to express the C-COL01191 clone *in vitro,* a plasmid was constructed according to the following method. First, the plasmid C-COL01191 was digested with restriction enzymes EcoRI and XbaI to collect DNA fragments of about 1.1 kb and about 2.0 kb. Next, a pcDNA3.1 (+) (Invitrogen) was digested with EcoRI and Xbal, to which the above-digested C-COL01191 fragment of about 2.0 kb was inserted. This ligation mixture was transformed into JM109 to obtain a plasmid. Next this plasmid was digested with EcoRI again, to which the above-digested C-COL01191 fragment of about 1.1 kb was inserted and transformed into JM109. The resultant plasmid was used as pIVCOL19 in the following reaction.

In vitro translation was conducted using TNT® Quick Coupled Transcription/Translation systems and Transcend™ Biotinylated tRNA (Promega) according to the provider's protocol. That is, 20 µl of TNT® Quick Master Mix, 0.5 µl of 1mM Methionine, 1.0 µl of Transcend™ Biotinylated tRNA and 0.5 µg of pIVCOL19 were mixed and a nuclease-free water was added such that a final volume became 25 µl and then the reaction was conducted at 30°C for 90 minutes. Thereafter 30µl of ElectroPure Reagents Tris-SDS Sample Buffer 50ml 2xConcentrate (Daiichi Pure Chemicals Co. Ltd.) was added to stop the reaction. Next 17 µl of the reaction liquid was subjected to electrophoresis on SDS-PAGE to electrically transfer onto a PVDF membrane and then reacted with a HRP-conjugated streptavidine (Rosche Diagnostic). The detection was conducted using ECL Western Blotting Detection System (Amersham Pharmacia Biotech). As the result, the expression of the protein was confirmed at the position of about 60 kDa.

Example 4: Determination of activity

Activity of inhibiting adhesion of monocytes to vascular endothelial cells

Vascular endothelial cells seeded in a culture plate is incubated in a medium under 5% $CO_2$ and 37°C. The cells are grown to a confluent condition, a recombinant "C-COL01191" protein is added and incubated. Thereafter a human recombinant TNF-α is added and cultured. A control group in which "C-COL01191" protein is not added (only the stimulation with TNF-α) is provided. To the thus-treated vascular endothelial cells is added fluorescence-labeled THP-1 cells and incubated at 37°C. Non-adhesion cells are removed by centrifugation. A lysis buffer is added to the well for the lysis of the cells. A fluorescence intensity at excitation wavelength of 485 nm and emission wavelength of 535 nm is determined. It is confirmed that the fluorescence intensity in the group with addition of the "C-COL01191" protein is significantly lower than that in the control group. Consequently, it can be supported that the "C-COL01191" protein has an activity of inhibiting the adhesion of monocytes to vascular endothelial cells.

Activity of inhibiting proliferation of vascular smooth muscle cells

Arterial smooth muscle cells were seeded in a plastic plate and cultured in a medium containing FBS in a semiconfluent condition at 37°C in 5% $CO_2$ and 95% air. DNA synthesis by arterial smooth muscle cells were determined in terms of an amount of [$^3$H]-thymidine incorporated. That is, the cells inoculated in the plastic plate were treated with the "C-COL01191" protein and/or a cell growth factor such as HB-EGF or the like for control in the medium for 24 hours. Next, [$^3$H]-thymidine was added to cultures. The cells were washed with PBS twice and treated with an ice-cooled trichloroacetic acid solution. Then, a lysis buffer was added for the lysis of the cells. A radioactivity in the cell lysate was determined by using liquid scintillation counter. The group with the addition of the "C-COL01191" protein shows the radioactivity significantly lower than that in the control group. Consequently, it became clear that the "C-COL01191" protein has an activity of inhibiting the proliferation of vascular smooth muscle cells.

Example 5: Preparation of "C-COL01191" protein of soluble type

In order to use as an administering antigen for producing an antibody, a chimeric protein comprising a hexahistidine tag fused to the C-terminal of Ser corresponding to No. 546 amino acid of the "C-COL01191" protein (see SEQ ID No:3) (hereinafter referred to "COL01191-His) was produced. A COL01191-His expression plasmid was constructed according to the following method. That is, the plasmid C-COL01191 was digested with BamHI and HincII and subjected to electrophoresis on agarose gel to collect a DNA fragment of about 1.7 kb. While, a pcDNA3.1/Myc-His C (Invitrogen) was digested with BamHI and EcoRV and then ligated with the above DNA fragment. A competent cell JM109 was transformed according to a routine method to prepare a COL01191-His expression plasmid. The resultant expression plasmid was transfected into COS cells according to the following method. That is, 50 µl of PuGENE6 (Rosche Diagnostic) and 12.5 µg of each of the above plasmids were mixed according to the provider's protocol and added to COS cells grown in a semiconfluent condition in a 150 $cm^2$ flask. They were cultured at conditions of 5% $CO_2$ and 37°C for 3 days and then a culture supernatant was harvested. The supernatant contained the COL01191-His in was subjected to SDS-PAGE elecrophoresis and then western blotting using an anti-His antibody (Penta.His™ Antibody; QIAGEN) and an anti-mouse immunoglobulins antibody conjugated with peroxidase (DAKO). As an administering antigen and a standard of ELISA system, the soluble type of the "C-COL01191" protein was expressed at large scale, and passed through a nickel column (Hitrap Chelating HP column; Amersham Biotechnology) to purify the "C-COL01191" protein of soluble type from the culture supernatant. After this protein was dialyzed against a 0.1M phosphate buffer (pH 7.4) (hereinafter abbreviated as "PBS"; Sigma), the concentration of the thus-purified "C-COL01191" protein of soluble type was calculated from an absorbance at 280 nm (extinction coefficient= 1 mg/ml).

Example 6: Production of "C-COL01191" protein specific polyclonal antibody

A rabbit was immunized with the "C-COL01191" protein (COL01191-His) prepared in Example 5 to produce a polyclonal antibody. That is, 20 µg of the "C-COL01191" protein and an equal amount of Freund's incomplete adjuvant (DIFCO) were mixed and 1 ml of the mixture was intradermally injected to a back of a female New Zealand white rabbit (Kitayama Labes Co., Ltd.; 2.0 to 2.49 kg). After two weeks, 20 µg of the "C-COL01191" protein and an equal amount of Freund's incomplete adjuvant (DIFCO) were mixed and the mixture was applied similarly. After additional one week, 20 µg of the "C-COL01191" protein was applied via auricular vein. After 2 months from the administration, a blood was taken via auricular vein and an antiserum was separated according to a routine method. Thereafter, the antibody was purified as follows. Ammonium sulfate was. added to the antiserum such that a final concentration became 33% and stirred at 4°C for 1 hour. The resultant precipitates were centrifuged. Then the precipitates were dissolved in PBS (pH 7.4), dialyzed and then only IgG fraction was purified through Procep A column (Millipore). This purified antibody is referred to "AD1 antibody" hereinafter. A pH of the eluate was returned to neutral and then dialyzed against PBS. A concentration of the protein was calculated from an absorbance at 280 nm (extinction coefficient= 0.533 mg/ml).

Example 7: Production of "C-COL01191" protein specific monoclonal antibody

100 µg of the "C-COL01191" protein (COL01191-His) was dissolved in 100 µl of a saline and mixed with an equal amount of Freund's complete adjuvant (DIFCO). 100 µl of the mixture was injected to each hind pad of a Wister female rat of 8 week-old. After 2 weeks, an iliac lymph node was extracted to be used for cell fusion. That is, lymphocytes were separated from the lymph node using a cell strainer (Farcon), mixed with myeloma cells (Sp2/O-Ag14) and then they were subjected to cell fusion using polyethylene glycol according to the method described in "Introduction of Experimental Procedures of Monoclonal antibodies", written by Tamie ANDOH and Joh CHIBA, p. 83, published by Kodansha Ltd. (1991). Hybridomas were selected using a HAT medium. After one week, a hybridoma producing the desired antibody was screened.

Screening was conducted according to an ELISA method directly immobilizing the "C-COL01191" protein (COL01191-His) on a plate. First, 50 µl of the "C-COL01191" protein or the different His-tag protein diluted to 1 µg/ml with PBS was added to each well of an immunoplate (Maxisorp; NUNC) and allowed to stand at 4°C overnight. Next, the plate was washed with an ion-exchanged water five times and then 100 µl of PBS containing 0.1% of BSA was added to each well and allowed to stand at room temperature for 1 hour so as to be blocked. A culture supernatant taken from the resultant hybridoma was added to each well and reacted at 37°C for 1 hour. Thereafter, the plate was washed with a saline containing 0.05% of Tween 20 three times. Next, 50 µl of a solution of an anti-rat immunoglobulins antibody conjugated with peroxidase (DAKO) diluted with PBS containing 10% of a rabbit serum 1000 times was added to each well. After the reaction was conducted at 37°C for 1 hour, the plate was washed similarly five times and a solution of tetramethylbenzidine (TMB.BioFX) was added to each well. After the reaction was conducted at room temperature for 10 minutes, a 0.5M sulfuric acid solution was added to stop the reaction. An absorbance at 450 nm was determined in a plate spectrophotometer (NJ-2100; Nippon Intermed). Thus, a well containing a hybridoma which produced an antibody reacting with the "C-COL01191" protein, but not reacting with the different His-tag protein was selected and the hybridoma was cloned according to a limiting dilution method. After 10 days, a hybridoma which produced an antibody binding to the "C-COL01191" protein was similarly screened. As the result, a monoclonal antibody, F1112-3-1 antibody, binding to only the "C-COL01191" protein was obtained. This hybridoma was cultured in a 10% FCS/RPMI-1640 medium (GIBCO) and then a Hybridoma-SFM medium (GIBCO) to produce an antibody. The antibody was purified using a Prosep-G column (Bioppressessing).

Example 8: Preparation of sandwich ELISA system

A sandwich ELISA system was prepared using an AD1 antibody immobilized solid phase/an AD1 antibody conjugated with peroxidase as follows: First, 0.5 mg of peroxidase (Toyobo Co., Ltd.) was dissolved in a distilled water according to the method of Nakane et al. (J. Histochem. Cytochem., Vol. 22, p. 1084 (1974)), to which 100mM periodic acid dissolved in a distilled water was added and reacted at 25°C for 20 minutes. After the reaction was finished, 1.5% ethyleneglycol was added and reacted at 25°C for 10 minutes. Thereafter, the reaction was dialyzed against an 1mM acetate buffer (pH 4.4). The thus-purified AD1 antibody was dialyzed against a 10mM carbonate buffer (pH 9.5). 0.5 mg of peroxidase activated by adding an 1M carbonate buffer (pH 9.5) was mixed with an equal amount of each antibody and reacted at 25°C for 2 hours. Further, 4 mg/ml of sodium borohydride was added and reacted at 4°C for 2 hours. The reaction was dialyzed against PBS to obtain a peroxidase-conjugated AD1 antibody. A plate on which an Ad1 antibody was immobilized was prepared as follows: First an Ad1 antibody was diluted with SCB (pH 9.5) to 20 μg/ml and 50 μl of the thus-diluted antibody was added to each well of an immunoplate (Maxisorp; NUNC). It was reacted at 56°C for 30 minutes and then washed with an ion-exchanged water five times. To each well was added 100 μl of PBS (pH 7.4) containing 20% blockace (Snow Brand Milk Products Co., Ltd.), thereby the Ad1 antibody solid phase plate was prepared. Standards were prepared by diluting the purified "C-COL01191" protein with PBS (pH 7.4) containing 0.1% BSA to 15.6, 31.3, 62.5, 125, 250, 500 and 1000 ng/ml. As a blank, PBS (pH 7.4) containing 0.1% BSA was used. For determination, the blocking agent of the plate was decated and each 50 μl of the above-prepared standard and the blank were poured and reacted at 4°C overnight. Next, the plate was washed with a saline containing 0.05% Tween 20 five times, and 50 μl of the peroxidase-conjugated AD1 antibody diluted to 0.5 μg/ml with PBS (pH 7.4) containing 1% BSA and 0.1% Tween 20 was poured and reacted at 37°C for 1 hour. Washed with a saline containing 0.05% Tween 20 five times, a tetramethylbenzidine (TMB.BioFX) was added to each well. The reaction was conducted at room temperature for 20 minutes and then stopped by adding a 0.5M sulfuric acid solution. An absorbance at 450 nm was determined in a plate spectrophtometer (NJ-2100; Nippon Intermed) to prepare a standard curve as shown in Fig. 2.

Example 9: Determination of "C-COL01191" protein in sera of various patients

20 Healthy subjects (10 males and 10 females) and 100 patients suffering from various diseases (34 diseases) were tested using the assay method system described in Example 8. As shown in Fig. 3, the determined values of the healthy subjects ranged 1.5 to 2.5 μg/ml. No difference between sexes was found. Diseases having a tendency to show a lower value were coronary diseases (angina pectoris, acute myocardial infarction), type II diabetes, pulmonary tumor, heart failure, cardiac disease, hypertension, hepatic failure, hepatic cirrhosis, cholelith, pancreatitis, acute leukemia, chronic leukemia, cerebral infarction, senile dementia Alzheimer-type, dementia and Sjogren's syndrome. The fact that cardiovascular diseases show lower values was similar to adiponectin. Whereas, in ovarian cancer, autoimmune hepatitis, osteoporosis, Cushing's disease and rheumatoid arthritis clearly the "C-COL01191" protein showed higher value. A diseases showing a high adiponectin value has not been reported. Therefore, the higher values shown in these diseases is said to be inherent in the "C-COL01191" protein.

The "C-COL01191" protein is a protein detected in blood of healthy subjects at a concentration as high as the order of 1.5 to 2.5 μg/ml and patients suffering from coronary diseases, diabetes and the like have a tendency to show the "C-COL01191" protein values lower than those of healthy subjects, while patients suffering from ovarian cancer, autoimmune hepatitis, osteoporosis, Cushing's disease and rheumatoid arthritis have a tendency to show the "C-COL01191" protein values higher than those of healthy subjects. Thus, it became clear that the "C-COL01191" protein is related to the above diseases.

The following table summarizes diseases showing higher and lower determined values of the "C-COL01191" protein.

| | normal range | diseases having tendency to show higher value | diseases having tendency to show -lower value |
|---|---|---|---|

(continued)

| | | | |
|---|---|---|---|
| "C-COL01191" | 1.5 to 2.5 μg/ml | ovarian cancer, autoimmune, hepatitis, osteoporosis, Cushing's disease, rheumatism | coronary diseases (angina pectoris, acute myocardial infarction), type II diabetes, pulmonary tumor, heart failure, cardiac disease, hypertension, hepatic failure, hepatic cirrhosis, cholelith, pancreatitis, acute/chronic leukemia, cerebral infarction, Alzheimer's disease, dementia, Sjogren's syndrom |

EFFECT OF THE INVENTION

The present gene is a gene encoding an adiponectin-like protein. This protein can be useful as a therapeutic agent for diseases in which the protein of the present invention is involved such as arteriosclerosis and the like. A nucleic acid of the above gene or a vector expressing this nucleic acid can be also useful as a therapeutic agent for diseases in which the protein of the present invention is involved such as arteriosclerosis and the like. Further, the present gene can be used as a diagnostic probe. That is, the present gene is useful in investigation, prevention and treatment of diseases in which the protein of the present invention is involved such as arteriosclerosis and the like.

**Claims**

1. The following DNA (a) or (b):

   (a) a DNA comprising the nucleotide sequence represented by SEQ ID NO:1, or
   (b) a DNA hybridizable with the DNA of SEQ ID NO:1 under stringent conditions and encoding a protein having an activity of inhibiting the proliferation of vascular smooth muscle cells.

2. A protein encoded by the DNA as defined in claim 1 or a salt thereof.

3. The following protein (a) or (b), or a salt thereof:

   (a) a protein comprising the amino acid sequence represented by SEQ ID NO:3, or
   (b) a protein comprising an amino acid sequence wherein deletion, substitution or addition of amino acid(s) has occurred in the amino acid sequence of SEQ ID NO:3 and having an activity of inhibiting the proliferation of vascular smooth muscle cells.

4. A recombinant vector including the DNA as defined in claim 1.

5. A transformant produced by transformation with the recombinant vector as defined in claim 4.

6. An antibody against the protein as defined in claim 2 or 3 or its partial peptide or a salt thereof.

7. A method of screening an agent capable of controlling the activity of the protein as defined in claim 2 or 3 among candidates, which comprises contacting the protein or the transformant as defined in claim 5 expressing the protein with the candidates and then detecting a change in activity of the protein.

8. A method of screening an agent capable of controlling the expression of the DNA as defined in claim 1 among candidates, which comprises contacting the vector as defined in claim 4 or the transformant as defined in claim 5 with the candidates and then detecting a change in expression level of the DNA as defined in claim 1.

9. A pharmaceutical composition comprising the protein as defined in claim 2 or 3 or a salt thereof.

10. An antisense nucleic acid inhibiting the expression of the protein as defined in claim 2 or 3.

11. An antisense nucleic acid as defined in claim 10 whose nucleotide sequence is a complementary sequence to the entire or a part of the DNA as defined in claim 1.

12. An assay method for the protein as defined in claim 2 or 3 in a test sample, which comprises using the antibody as defined in claim 6.

13. An assay reagent or kit for the protein as define in claim 2 or 3 in a test sample, which comprises using the antibody as defined in claim 6.

Fig. 1

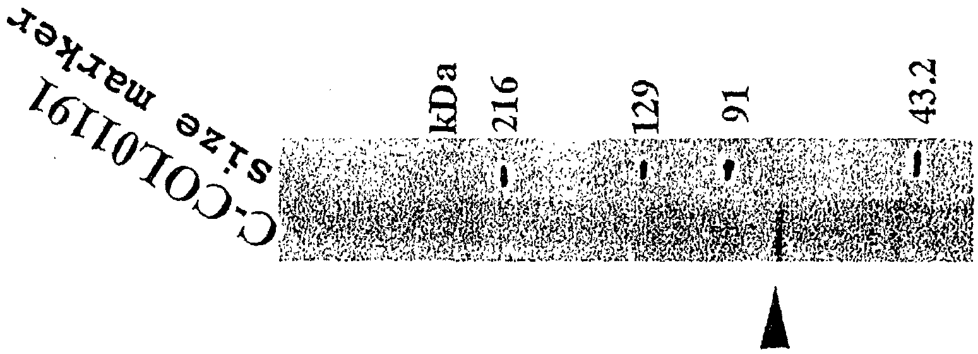
C-COL01191
size marker
kDa
216
129
91
43.2

Fig. 2

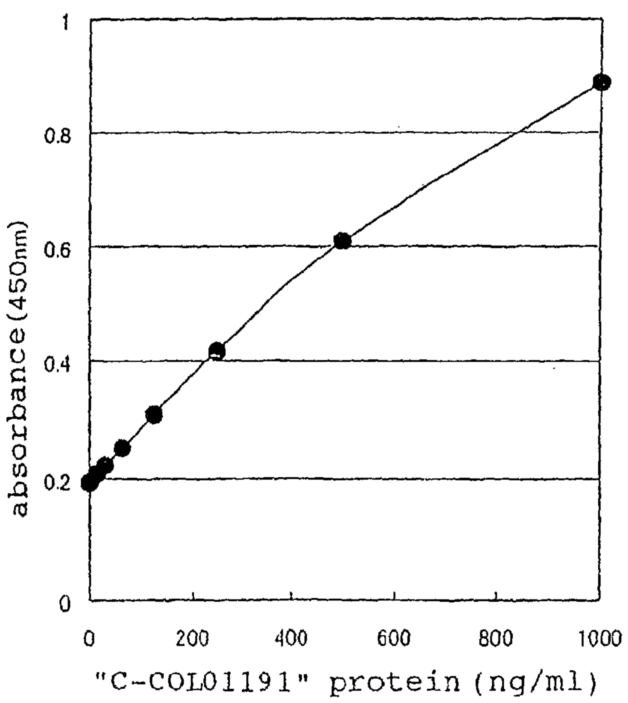
absorbance(450nm)
0
0.2
0.4
0.6
0.8
1
0
200
400
600
800
1000
"C-COL01191" protein (ng/ml)

Fig. 3

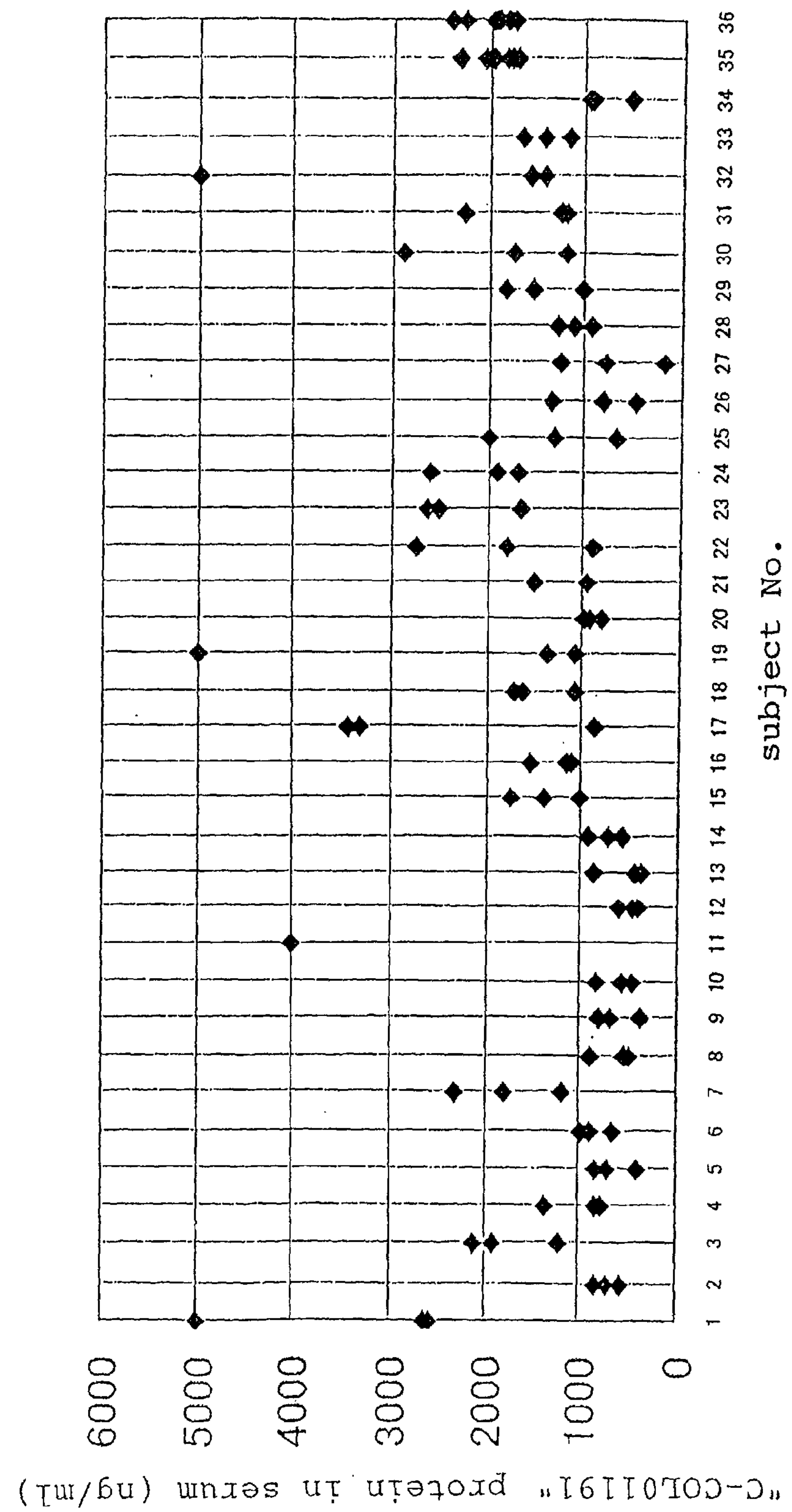
"C-COL01191" protein in serum (ng/ml)
6000
5000
4000
3000
2000
1000
0
subject No.
1 2 3 4 5 6 7 8 9 10 11 12 13 14 15 16 17 18 19 20 21 22 23 24 25 26 27 28 29 30 31 32 33 34 35 36

Fig. 3 (continued)

| subject No. | target disease | name of disease |
|---|---|---|
| 1 | cancers | ovarian cancer |
| 2 | | pulmonary tumor |
| 3 | | prostatic cancer |
| 4 | cardiovascular diseases | angina pectoris |
| 5 | | acute myocardial infarction |
| 6 | | heart failure |
| 7 | | arrhythmia |
| 8 | | cardiac disease |
| 9 | | hypertension |
| 10 | hepatopathy/ cholecystopathy/ pancreatopathy | hepatic failure |
| 11 | | autoimmune hepatitis |
| 12 | | hepatic cirrhosis |
| 13 | | choleith |
| 14 | | pancreatitis |
| 15 | metabolic/trophic diseases | type II diabetes |
| 16 | | hyperlipemia |
| 17 | | osteoporosis |
| 18 | endocrinological diseases | Graves's disease |
| 19 | | Cushing's syndrome |
| 20 | blood diseases | acute leukemia |
| 21 | | chromic leukemia |
| 22 | | myeloma |
| 23 | nephropathy/ uropathy | kidney failure |
| 24 | | glomerular nephritis |
| 25 | | prostatic hyperplasia |
| 26 | neuropathy | cerebral infarction |
| 27 | | senile dementia Alzheimer-type |
| 28 | | dementia |
| 29 | allergy | pollinosis |
| 30 | | atopic dermatitis |
| 31 | | bronchial asthma |
| 32 | immunodeficient/rheumatism | rheumatoid arthritis |
| 33 | | vasculitis syndrome |
| 34 | | Sjogren's syndrome |
| 35 | healthy | healthy female |
| 36 | | healthy male |

# INTERNATIONAL SEARCH REPORT

International application No.
PCT/JP02/00844

A. CLASSIFICATION OF SUBJECT MATTER
(See extra sheet.)

According to International Patent Classification (IPC) or to both national classification and IPC

B. FIELDS SEARCHED

Minimum documentation searched (classification system followed by classification symbols)
(See extra sheet.)

Documentation searched other than minimum documentation to the extent that such documents are included in the fields searched

Electronic data base consulted during the international search (name of data base and, where practicable, search terms used)
GenBank/EMBL/DDBJ/GeneSeq, SwissProt/PIR/GeneSeq, BIOSIS(DAILOG), WPI(DIALOG), MEDLINE(STN)

C. DOCUMENTS CONSIDERED TO BE RELEVANT

| Category* | Citation of document, with indication, where appropriate, of the relevant passages | Relevant to claim No. |
|---|---|---|
| Y/A | MAEDA, K. et al., cDNA cloning and expression of a novel adipose specific collagen-like factor, apM1 (AdiPose Most abundant Gene transcript 1). Biochem. Biophys.Res.Commun., 1996, Vol.221, No.2, p.286-9 | 3,6,9,12, 13/1,2,4, 5,7,8,10, 11 |
| Y/A | MATSUZAWA, Y. et al., Molecular mechanism of metabolic syndrome X: contribution of adipocytokines adipocyte-derived bioactive substances. Ann.N.Y.Acad.Sci.,1999,Vol.892, p.146-54 | 3,6,9,12, 13/1,2,4, 5,7,8,10, 11 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 April, 2002 (16.04.02) | 30 April, 2002 (30.04.02) |
| Name and mailing address of the ISA/ Japanese Patent Office | Authorized officer |
| Facsimile No. | Telephone No. |

Form PCT/ISA/210 (second sheet) (July 1998)

INTERNATIONAL SEARCH REPORT

International application No.
PCT/JP02/00844

Continuation of A. CLASSIFICATION OF SUBJECT MATTER
(International Patent Classification (IPC))

Int.Cl⁷ C12N15/09, A61K38/17, A61K48/00, A61P9/10, C07K14/47, C07K16/18, C12N1/15, C12N1/19, C12N1/21, C12N5/10, C12Q1/02, C12Q1/68, G01N33/15, G01N33/50, G01N33/53, C12P21/08//(C12Q1/02 C12R1:91)
(According to International Patent Classification (IPC) or to both national classification and IPC)

Continuation of B. FIELDS SEARCHED
Minimum Documentation Searched(International Patent Classification (IPC))

Int.Cl⁷ C12N15/09, A61K38/17, A61K48/00, A61P9/10, C07K14/47, C07K16/18, C12N1/15, C12N1/19, C12N1/21, C12N5/10, C12Q1/02, C12Q1/68, G01N33/15, G01N33/50, G01N33/53, C12P21/08//(C12Q1/02 C12R1:91)
Minimum documentation searched (classification system followed by classification symbols)

Form PCT/ISA/210 (extra sheet) (July 1998)